# FASCICULE DE BREVET EUROPEEN

(11) **EP 0 599 703 B1**
(45) Date de publication et mention de la délivrance du brevet: **17.09.1997**
(21) Numéro de dépôt: 93402807.7
(22) Date de dépôt: 19.11.1993
(51) Int. Cl.: A61K 7/13

(54) **Utilisation du 4-hydroxy- ou 4-aminobenzimidazole ou de leurs dérivés comme coupleurs dans des compositions tinctoriales d'oxydation, compositions et procédés de mise en oeuvre**
Verwendung von 4-Hydroxy oder 4-Aminobenzimidazol oder Ihren Derivaten as wie Kuppler-substanzen in Oxidationsfärbungszusammensetzungen, sowie Zusammensetzungen und Verfahren zu deren Anwendung
Use of 4-hydroxy- or 4-aminobenzumidazol or their derivatives as coupler agent in oxidative dye compositions, the compositions and its method of application

(30) Priorité: 20.11.1992 FR 9213999
(43) Date de publication de la demande: 01.06.1994
(73) Titulaire: L'OREAL, 75008 Paris (FR)
(72) Inventeur: Audousset, Marie-Pascale, F-92300 Levallois-Perret (FR); Cotteret, Jean, F-78480 Verneuil-sur-Seine (FR)
(74) Mandataire: Casalonga, Axel

(56) Documents cités:
- EP-A- 0 004 368
- DE-A- 192 191

## Description

La présente invention est relative à l'utilisation du 4-hydroxy- ou 4-aminobenzimidazole ou de leurs dérivés comme coupleurs, en présence de précurseurs de colorants d'oxydation de type paraphénylènediamines dans des compositions tinctoriales pour fibres kératiniques et en particulier pour cheveux humains, aux procédés de mise en oeuvre et aux compositions contenant le coupleur et au moins une p-phénylènediamine.

Il est connu de teindre les fibres kératiniques et en particulier les cheveux humains avec des compositions tinctoriales contenant des précurseurs de colorants d'oxydation et en particulier des p-phénylène diamines, des ortho- ou para-aminophénols appelés généralement "bases d'oxydation".

On sait également que l'on peut obtenir une coloration ou faire varier les nuances obtenues avec ces bases d'oxydation en les associant à des coupleurs encore appelés modificateurs de coloration, choisis notamment parmi des métadiamines aromatiques, des métaaminophénols et des métadiphénols.

On a également déjà envisagé, et notamment dans le brevet DE-A-192 19 11, l'utilisation à titre de coupleur de dérivés du benzimidazole avec des précurseurs de colorants d'oxydation, le précurseur de colorant d'oxydation ou "base" et le coupleur étant utilisés dans un rapport base/coupleur égal ou inférieur à 1.

On recherche dans le domaine de la coloration des cheveux, des compositions permettant de conférer à ceux-ci une coloration et une résistance satisfaisantes à la lumière, aux lavages, aux intempéries et à la transpiration.

La résistance à la transpiration présente une importance toute particulière, dans la mesure où les nuances bleues et cendrées ont souvent tendance à rougir sous l'action de la transpiration.

La demanderesse a découvert, de façon surprenante, qu'en utilisant certains dérivés de benzimidazole avec des bases d'oxydation choisies parmi les p-phénylènediamines et dans un rapport base d'oxydation/benzimidazole égal ou supérieur à 1,2 et de préférence égal ou supérieur à 1,5, il était possible d'obtenir des colorations présentant une ténacité particulièrement remarquable à la transpiration, tout en conservant une bonne résistance à la lumière et au lavage.

L'invention a donc pour objet l'utilisation du 4-hydroxy- ou du 4-aminobenzimidazole ou de leurs dérivés comme coupleurs dans des compositions tinctoriales pour fibres kératiniques, contenant des p-phénylènediamines, dans des rapports p-phénylènediamine/coupleur particuliers.

Un autre objet de l'invention est constitué par les compositions contenant le 4-hydroxy- ou le 4-aminobenzimidazole ou leurs dérivés et une p-phénylènediamine dans ces rapports particuliers.

L'invention a également pour objet un procédé de teinture mettant en oeuvre le 4-hydroxy- ou le 4-aminobenzimidazole ou leurs dérivés dans les proportions particulières, par rapport aux p-phénylène diamines.

D'autres objets de l'invention apparaîtront à la lecture de la description et des exemples qui suivent.

L'invention a donc pour objet l'utilisation d'un 4-hydroxy- ou d'un 4-aminobenzimidazole ou de leurs dérivés, répondant à la formule (I) : 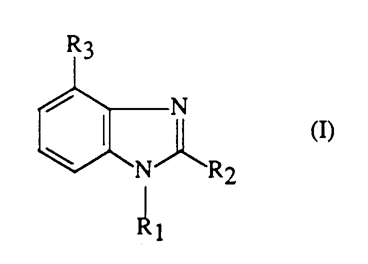 dans laquelle :
R₁ et R₂ représentent hydrogène ou alkyle, au moins un des substituants étant égal à hydrogène;
R₃ désigne OH ou NH₂,
à titre de coupleurs, pour la coloration des fibres kératiniques, en association avec une p-phénylènediamine, dans un rapport p-phénylènediamine/coupleur, égal ou supérieur à 1,2.

Le radical alkyle pour les composés de formule (I), désigne plus particulièrement alkyle inférieur ayant 1 à 4 atomes de carbone, tel que méthyle, éthyle, propyle.

Les paraphénylènediamines sont choisies parmi les composés répondant à la formule (II) ci-après : 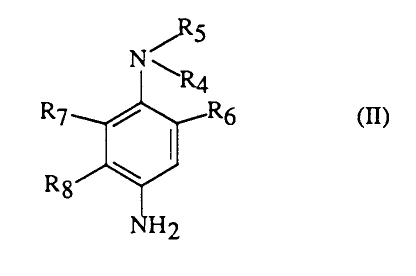 dans laquelle :
R₆, R₇ et R₈, identiques ou différents, représentent un atome d'hydrogène ou d'halogène, un radical alkyle ou hydroxyalkyle ayant 1 à 4 atomes de carbone, un radical alcoxy ayant 1 à 4 atomes de carbone; un radical carboxy; R₄ et R₅, identiques ou différents, représentent un atome d'hydrogène, un radical alkyle, hydroxyalkyle, alcoxyalkyle, carbamylalkyle, mésylaminoalkyle, acétylaminoalkyle, sulfoalkyle, uréidoalkyle, carbalcoxyaminoalkyle, pipéridinoalkyle, morpholinoalkyle, phényle; ces groupes alkyle ou alcoxy ayant de 1 à 4 atomes de carbone, ou bien R₄ et R₅ forment, conjointement avec l'atome d'azote auquel ils sont liés, un hétérocycle pipéridino ou morpholino, sous réserve que R₇ ou R₆ représente un atome d'hydrogène lorsque R₄ et R₅ ne représentent pas un atome d'hydrogène, ainsi que les sels de ces composés.

Parmi les composés de formule (II), on peut citer la p-phénylène diamine, la p-toluylènediamine, la méthoxyparaphénylènediamine, la chloroparaphénylènediamine, la 2,6-diméthylparaphénylènediamine, la 2,6-diéthylparaphénylènediamine, la 2,5-diméthylparaphénylène diamine, la 2-méthyl 5-méthoxyparaphénylènediamine, la 2,6-diméthyl 5-méthoxyparaphénylènediamine, la N,N-diméthylparaphénylènediamine, la N,N-diéthylparaphénylènediamine, la N,N-dipropylparaphénylènediamine, la 3-méthyl 4-amino N,N-diéthylaniline, la N,N-di (β-hydroxyéthyl)paraphénylènediamine, la 3-méthyl 4-amino N,N-di-(β-hydroxyéthyl)aniline, la 3-chloro 4-amino N,N-di-(β-hydroxyéthyl) aniline, la 4-amino N,N-(éthylcarbamylméthyl)aniline, la 3-méthyl 4-amino N',N-(éthylcarbamylméthyl)aniline, la 4-amino N,N-(éthyl,β-pipéridinoéthyl)aniline, la 3-méthyl 4-amino N,N-(éthyl,β-pipéridino éthyl)aniline, la 4-amino N,N-(éthyl,β-morpholinoéthyl)aniline, la 3-méthyl 4-amino N,N-(éthyl,β-morpholinoéthyl)aniline, la 4-amino N,N- (éthyl,β-acétylaminoéthyl)aniline, la 4-amino N-(β-méthoxyéthyl)aniline, la 3-méthyl 4-amino N,N-(éthyl,β-acétylaminoéthyl) aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino)phényl]morpholine, l aN-[(4'-amino) phényl]pipéridine, la 2-hydroxyéthylparaphénylènediamine, la fluoroparaphénylènediamine, la carboxyparaphénylènediamine, la 2-isopropylparaphénylènediamine, la 2-n-propylparaphénylènediamine, l'hydroxy-2-n-propylparaphénylènediamine, la 2-hydroxyméthylparaphénylènediamine.

Ces p-phénylènediamines peuvent être introduites dans la composition tinctoriale, soit sous forme de base libre, soit sous forme de sels, tels que sous forme de chlorhydrate, de bromhydrate ou de sulfate.

Selon une réalisation préférée, on utilise le 4-hydroxybenzimidazole que l'on associe à la p-phénylènediamine ou à la p-toluylènediamine.

En plus du coupleur de formule (I) et des p-phénylènediamines, on peut utiliser conjointement d'autres précurseurs de colorants de type para et/ou ortho, choisis plus particulièrement parmi les bases dites "doubles" qui sont des bis-phénylalkylènediamines, les précurseurs hétérocycliques para comme la 2,5-diaminopyridine, la 2-hydroxy 5-aminopyridine, la tétraaminopyrimidine et les orthoaminophénols comme le 1-amino 2-hydroxybenzène, le 6-méthyl 1-hydroxy 2-aminobenzène, le 4-méthyl 1-amino 2-hydroxybenzène et les orthophénylènediamines.

Les bases dites doubles de la famille des bis-phénylalkylènediamines, répondent à la formule : 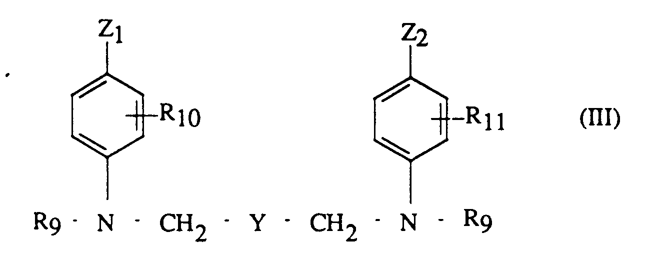 dans laquelle :
Z₁ et Z₂, identiques ou différents, représentent des groupements hydroxyle ou NHR₁₂, où R₁₂ désigne un atome d'hydrogène ou un radical alkyle inférieur;
R₁₀ et R₁₁, identiques ou différents, représentent soit des atomes d'hydrogène, soit des atomes d'halogène, soit encore des groupements alkyle;
R₉ représente un atome d'hydrogène, un groupe alkyle, hydroxyalkyle ou aminoalkyle, dont le reste amino peut être substitué;
Y représente un radical pris dans le groupe constitué par les radicaux suivants : -(CH₂)ₙ-, (CH₂)ₘ -O-(CH₂)ₘ, 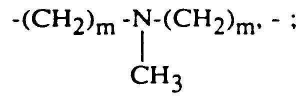 -(CH₂)ₘ-CHOH-(CH₂)ₘ;
n étant un nombre entier compris entre 0 et 8 et m un nombre entier compris entre 0 et 4, cette base pouvant se présenter sous forme de ses sels d'addition avec des acides.

Les radicaux alkyle ou alcoxy désignent de préférence un groupement ayant 1 à 4 atomes de carbone et notamment méthyle, éthyle, propyle, méthoxy, éthoxy.

Parmi les composés de formule (III), on peut citer le N,N'-bis-(β-hydroxyéthyl)N,N'-bis-(4'-aminophény)1,3-diamino 2-propanol, la N,N'-bis-(β-hydroxyéthyl)N,N'-bis-(4'-aminophényl)éthylènediamine, la N,N'-bis-(4-aminophényl)tétraméthylènediamine, la N,N'-bis-(β-hydroxyéthyl)N,N'-bis-(4-aminophényl)tétraméthylènediamine, la N, N'-bis-(4-méthylaminophényl)tétraméthylènediamine, la N,N'-bis-(éthyl)N,N'-bis-(4'-amino 3'-méthylphényl)éthylènediamine.

Une forme de réalisation préférée de l'invention consiste à utiliser au moins une paraphénylènediamine éventuellement associée à une base dite "double". On utilisera plus particulièrement le 4-hydroxybenzimidazole associé à la p-phénylènediamine ou à la p-toluylène diamine et au N,N'-bis-(β-hydroxyéthyl)N,N'-bis-(4'-aminophényl)1,3-diamino 2-propanol.

En plus du 4-hydroxy ou du 4-aminobenzimidazole ou de leurs dérivés, on peut utiliser conjointement d'autres coupleurs connus en eux-mêmes, tels que les métadiphénols, les métaaminophénols, les métaacylaminophénols, les métauréidophénols, les métacarbalcoxyaminophénols, les naphtols, les coupleurs possédant un groupement méthylène actif tel que les composés β-cétoniques, les pyrazolones ou encore les coupleurs de la famille des indoles décrits plus particulièrement dans les brevets suivants : FR-A-2.636.236, EP-A-428.442, EP-A-428.441, EP-A-496.653, EP-A-424.261, ainsi que le 4-hydroxyindole.

Les coupleurs particulièrement préférés sont choisis parmi le métaaminophénol, le 1,3-dihydroxy 4-chlorobenzène, le 1,3-dihydroxy benzène, l'α-naphtol, la 6-hydroxybenzomorpholine, le 1-méthyl 2-hydroxy 4-aminobenzène, le 1-méthyl 2-hydroxy 4-(2-hydroxyéthyl) aminobenzène, le 1,3-dihydroxy 2-méthylbenzène, le 1-hydroxy 3,4-méthylènedioxybenzène, le 1-(β-hydroxyéthylamino)-3,4-méthylène dioxybenzène, le 2-bromo 4,5-méthylènedioxyphénol, le 2-amino 5-acétamidophénol, le 6-hydroxyindole, le 7-hydroxyindole, le 7-aminoindole et le 4-hydroxyindole.

Il est également possible d'utiliser, conjointement avec les coupleurs de formule (I) et les p-phénylènediamines, des colorants directs bien connus dans l'état de la technique, notamment en vue de nuancer ou d'enrichir en reflets les colorations.

Ces colorants directs sont choisis en particulier parmi les colorants azoiques, anthraquinoniques ou les dérivés nitrés de la série benzénique.

La composition tinctoriale pour fibres kératiniques, en particulier pour cheveux humains, qui constitue un autre objet de l'invention, est essentiellement caractérisée par le fait qu'elle contient dans un milieu approprié pour la teinture des fibres kératiniques et cosmétiquement acceptable pour les cheveux, au moins un composé répondant à la formule (I) précitée, et au moins une p-phénylènediamine dans des proportions telles que le rapport p-phénylènediamine/coupleur de formule (I), soit égal ou supérieur à 1,2 et de préférence égal ou supérieur à 1,5.

Les p-phénylènediamines sont de préférence choisies parmi celles de formule (II) précitée, elles peuvent être associées à des bases doubles et la composition peut contenir d'autres coupleurs tels que définis ci-dessus et éventuellement des colorants directs.

Le coupleur de formule (I) est présent dans la composition dans des proportions suffisantes pour développer en milieu oxydant une coloration avec la p-phénylènediamine et de préférence comprise entre 0,008 et 3,5% en poids par rapport au poids total de la composition et en particulier comprise entre 0,05 et 2%.

La p-phénylènediamine est généralement présente dans des proportions comprises entre 0,01 et 10% en poids par rapport au poids total de la composition et en particulier entre 0,05 et 4%.

La coloration des fibres kératiniques et en particulier des cheveux humains est effectuée par la mise en oeuvre du coupleur de formule (I) et d'une p-phénylènediamine en présence d'un agent oxydant. Cet agent oxydant peut être choisi parmi le peroxyde d'hydrogène, le peroxyde d'urée, les bromates de métaux alcalins, les persels tels que les perborates et les persulfates. Le peroxyde d'hydrogène étant particulièrement préféré.

Le pH de la composition appliquée sur les fibres kératiniques et en particulier les cheveux, a une valeur généralement comprise entre 3 et 11.

Ce pH peut être ajusté par l'utilisation d'agents acidifiants ou alcalinisants bien connus dans le domaine de la teinture des fibres kératiniques et en particulier des cheveux.

Les compositions tinctoriales conformes à l'invention peuvent également contenir dans la forme de réalisation préférée, des agents tensio-actifs anioniques, cationiques, non-ioniques, amphotères ou leurs mélanges.

Ces agents tensio-actifs sont présents dans les compositions conformes à l'invention dans des proportions généralement compnses entre 0,5 et 55% en poids et de préférence entre 2 et 50% en poids par rapport au poids total de la composition.

Ces compositions peuvent également contenir des solvants organiques pour solubiliser les composés qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols, comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol et les produits analogues ou leurs mélanges.

Les solvants sont présents de préférence dans des proportions comprises entre 1 et 40% en poids, et en particulier entre 5 et 30% en poids par rapport au poids total de la composition.

Les agents épaississants que l'on peut ajouter dans les compositions conformes à l'invention peuvent être choisis parmi l'alginate de sodium, la gomme arabique, les dérivés de cellulose, les polymères d'acide acrylique, la gomme de xanthane, les scléroglucanes. On peut également utiliser des agents épaississants minéraux, tels que la bentonite.

Ces agents épaississants sont présents de préférence dans des proportions compnses entre 0,1 et 5%, et en particulier entre 0,2 et 3% en poids par rapport au poids total de la composition.

Les agents anti-oxydants qui peuvent être présents dans les compositions sont choisis en particulier parmi le sulfite de sodium, l'acide thioglycolique, le bisulfite de sodium, l'acide déhydroascorbique, l'hydroquinone et l'acide homogentisique.

Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquides, de crèmes, de gels ou sous toute autre forme appropriée pour réaliser une teinture des fibres kératiniques et notamment des cheveux humains. Ces compositions peuvent être conditionnées en flacons aérosols en présence d'un agent propulseur.

Le procédé de teinture des fibres kératiniques, en particulier des cheveux, conforme à l'invention, consiste à appliquer sur les fibres kératiniques une composition préparée au moment de l'emploi et contenant au moins un coupleur de formule (I), au moins une p-phénylènediamine dans le rapport p-phénylènediamine/coupleur de formule (I) défini ci-dessus et au moins un agent oxydant dans une quantité suffisante pour pouvoir développer une coloration.

Selon un mode de réalisation particulier, on utilise une solution de peroxyde d'hydrogène dans une concentration de 5 à 40 volumes. Cette solution est additionnée à la composition tinctoriale et le mélange obtenu est appliqué sur les fibres kératiniques. On laisse poser pendant 5 à 40 minutes et de préférence 15 à 30 minutes, après quoi on rince les fibres, on les lave au shampooing, on les rince à nouveau et on les sèche.

Selon une autre forme de réalisation de l'invention, on applique séparément une composition (A) contenant dans un milieu cosmétiquement acceptable le coupleur de formule (I) et une p-phénylènediamine dans le rapport défini ci-dessus sur les fibres kératiniques, et après rinçage, une composition (B) renfermant l'agent oxydant.

Il est également possible, conformément à l'invention, d'appliquer séparément une composition contenant dans un milieu cosmétiquement acceptable une p-phénylènediamine, puis après rinçage, d'appliquer une composition renfermant le coupleur de formule (I) dans un milieu cosmétiquement acceptable, et dans une dernière étape, l'agent oxydant, la p-phénylènediamine et le coupleur étant appliqués dans des proportions telles que le rapport au niveau des fibres kératiniques entre la p-phénylènediamine et le coupleur de formule (I), soit supérieur ou égal à 1,2, de préférence supérieur ou égal à 1,5.

Une autre forme de réalisation consiste à appliquer dans un premier temps une composition contenant une p-phénylènediamine, puis après rinçage, une composition (B) renfermant dans un milieu cosmétiqúement acceptable, le coupleur de formule (I) et l'agent oxydant; la p-phénylènediamine et le coupleur de formule (I) étant mis en oeuvre dans les rapports définis ci-dessus.

Les exemples qui suivent sont destinés à illustrer l'invention.

Les marques commerciales mentionnées dans les exemples sont, selon la connaissance de la Demanderesse, des marques déposées.

### EXEMPLES 1 à 5

On procède à la teinture des cheveux en appliquant sur des cheveux gris à 90% de blancs ou sur des cheveux décolorés, un mélange extemporané, poids pour poids, de la composition colorante (A) et de la composition oxydante (B) (28 g du mélange pour 3 g de cheveux).

On laisse agir ce mélange pendant 30 minutes, puis on rince les cheveux, on effectue un shampooing. Après séchage, les cheveux sont teints dans la nuance précisée au bas du tableau.

| en g | 1 | 2 | 3 | 4 | 5 |
|---|---|---|---|---|---|
| **Composition colorante (A)** | | | | | |
| 4-hydroxybenzimidazole, HBr | 0,6 | 0,86 | 0,75 | 1,32 | 1,16 |
| 2,6-diméthylparaphénylène diamine, 2HCl | 0,8 | | | | |
| Paratoluènediamine, 2HCl | | 1,14 | 1 | 1,58 | 1,74 |
| Support 1 | X | | | | |
| Support 2 | | X | X | X | X |
| Eau qsp | 100 | 100 | 100 | 100 | 100 |
| **Rapport Base/Coupleur** | 1,33 | 1,32 | 1,34 | 1,2 | 1,5 |

| **Composition oxydante (B)** | | | | | |
|---|---|---|---|---|---|
| Solution d'eau oxygénée à 20 volumes | | | | | |
| Acide phosphorique qs pH | 1-1,5 | 3 | 3 | 3 | 3 |

| **Nuances obtenues :** | | | | | |
|---|---|---|---|---|---|
| sur cheveux naturels gris à 90 % blancs | bleu intense | bleu | bleu | - | - |
| sur cheveux décolorés | - | - | - | noir | noir |

### SUPPORT DE COLORATION 1

| | |
|---|---|
| - Alcool oléique polyglycérolé à 2 moles de glycérol | 4 g |
| - Alcool oléique polyglycérolé à 4 moles de glycérol à 78% de MA | 5,7 g MA |
| - Acide oléique | 3 g |
| - Amine oléique 2 OE, vendue sous la dénomination "ETHOMEEN O 12" par la Société AKZO | 7 g |
| - Laurylamino succinamate de diéthylaminopropyle, sel de sodium à 55% de MA | 3 g MA |
| - Alcool oléique | 5 g |
| - Diéthanolamide d'acide oléique | 12 g |
| - Propylèneglycol | 3,5 g |
| - Alcool éthylique | 7 g |
| - Dipropylèneglycol | 0,5 g |
| - Monométhyl éther de propylèneglycol | 9 g |
| - Métabisulfite de sodium en solution aqueuse à 35% de MA | 0,46 g MA |
| - Acétate d'ammonium | 0,8 g |
| - Antioxydant, séquestrant qs | |
| - Monoéthanolamine qs | pH=9,8 |

### SUPPORT DE COLORATION 2

| | |
|---|---|
| - Laurylsulfate d'ammonium à 30% de MA | 6 g MA |
| - Métabisulfite de sodium en solution aqueuse à 35% de MA | 0,35 g MA |
| - Ammoniaque en solution aqueuse à 20% de MA | 2 g MA |
| - Séquestrant qs | |

### EXEMPLE 6

| | |
|---|---|
| - 4-aminobenzimidazole | 1,16 g |
| - Paratoluylènediamine | 1,74 g |
| - Laurylsulfate d'ammonium | 20 g |
| - TRILON B | 0,3 g |
| - Ammoniaque à 20% | 10 g |
| - Bisulfite de sodium à 40% | 1 g |
| - Eau qsp | 100 g |

Le rapport en poids base/coupleur est égal à 1,5.

Au moment de l'emploi, cette composition est mélangée à un poids égal d'eau oxygénée à 20 volumes et est appliquée sur des mèches de cheveux gris permanentées.

Après un temps de pose de 30 minutes suivi d'un rinçage, les cheveux sont teints en brun.

### EXEMPLE 7

| | |
|---|---|
| - Bromhydrate de 4-hydroxybenzimidazole | 0,66 g |
| - Paratoluènediamine | 0,79 g |
| - Laurylsulfate d'ammonium | 6 g |
| - Acide éthylènediamine tétracétique | 0,3 g |
| - Ammoniaque à 20% | 10 g |
| - Métabisulfite de sodium | 0,35 g |
| - Eau qsp | 100 g |

Au moment de l'emploi, cette composition est mélangée poids pour poids avec une solution d'eau oxygénée à 20 volumes.

La composition résultante appliquée sur des cheveux gris à 90% de blancs, pendant 30 minutes, leur confère après rinçage et lavage une coloration gris foncé violine.

## Revendications

1. Utilisation du 4-hydroxy- ou du 4-aminobenzimidazole ou de leurs dérivés, répondant à la formule (I) : 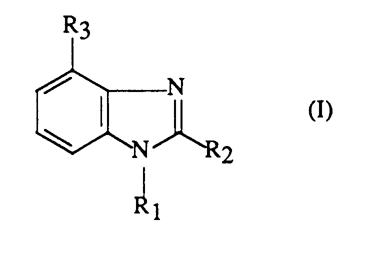 dans laquelle :
R₁ et R₂ représentent hydrogène ou alkyle; au moins un des substituants étant égal à hydrogène;
R₃ désigne OH ou NH₂, à titre de coupleur pour la teinture des fibres kératiniques, en particulier des cheveux, en présence d'une paraphénylènediamine, le rapport entre la paraphénylènediamine et le coupleur de formule (I) étant supérieur ou égal à 1,2.

2. Utilisation selon la revendication 1, caractérisée par le fait que la paraphénylènediamine est choisie parmi les composés répondant à la formule (II) : 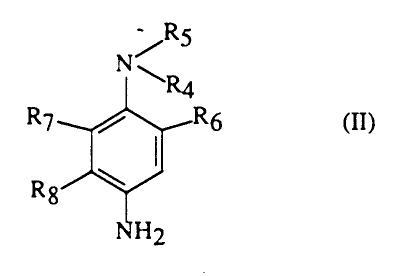 dans laquelle :
R₆, R₇ et R₈, identiques ou différents, représentent un atome d'hydrogène ou d'halogène, un radical alkyle ou hydroxyalkyle ayant 1 à 4 atomes de carbone, un radical alcoxy ayant 1 à 4 atomes de carbone, un radical carboxy; R₄ et R₅, identiques ou différents, représentent un atome d'hydrogène, un radical alkyle, hydroxyalkyle, alcoxyalkyle, carbamylalkyle, mésylaminoalkyle, acétylaminoalkyle, ulfoalkyle, uréidoalkyle, carbalcoxyaminoalkyle, pipéridinoalkyle, morpholinoalkyle, phényle ; ces groupes alkyle ou alcoxy ayant de 1 à 4 atomes de carbone, ou bien R₄ et R₅ forment, conjointement avec l'atome d'azote auquel ils sont liés, un hétérocycle pipéridino ou morpholino, sous réserve que R₆ ou R₇ représente un atome d'hydrogène lorsque R₄ et R₅ ne représentent pas un atome d'hydrogène, ainsi que les sels de ces composés.

3. Utilisation selon l'une des revendications 1 ou 2, caractérisée par le fait qu'on associe le 4-hydroxybenzimidazole à la p-phénylène diamine ou à la p-toluylènediamine.

4. Utilisation selon l'une quelconque des revendications 1 à 3, caractérisée par le fait qu'on utilise également d'autres précurseurs de colorants d'oxydation du type ortho choisis parmi les orthoaminophénols et les orthophénylènediamines, et du type para choisis parmi les bis-phénylalkylènediamines, les précurseurs hétérocycliques para.

5. Utilisation selon la revendication 4, caractérisée par le fait que les bases dites "doubles" de la famille des bis-phénylalkylènediamines sont choisies parmi les composés répondant à la formule (III) : 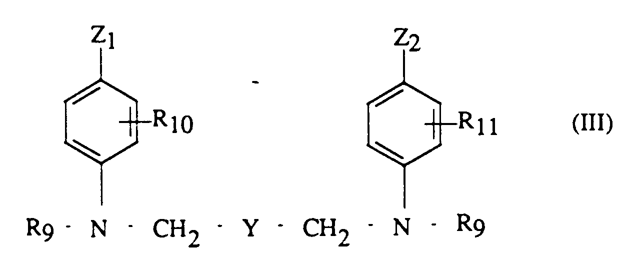 dans laquelle :
Z₁ et Z₂, identiques ou différents, représentent des groupements hydroxyle ou NHR₁₂, où R₁₂ désigne un atome d'hydrogène ou un radical alkyle inférieur;
R₁₀ et R₁₁, identiques ou différents, représentent soit des atomes d'hydrogène, soit des atomes d'halogène, soit encore des groupements alkyle;
R₉ représente un atome d'hydrogène, un groupe alkyle, hydroxyalkyle ou aminoalkyle, dont le reste amino peut être substitué;
Y représente un radical pris dans le groupe constitué par les radicaux suivants : -(CH₂)ₙ-, (CH₂)ₘ-O-(CH₂)ₘ, 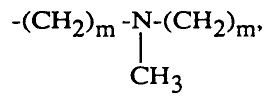 -et -(CH₂)ₘ-CHOH-(CH₂)ₘ ;
n étant un nombre entier compris entre 0 et 8 et m un nombre entier compris entre 0 et 4, cette base pouvant se présenter sous forme de ses sels d'addition avec des acides.

6. Utilisation selon l'une quelconque des revendications 1 à 5, caractérisée par le fait que l'on associe le 4-hydroxybenzimidazole à la p-phénylènediamine ou à la p-toluylènediamine et au N,N'-bis-(β-hydroxyéthyl)N,N'-bis-(4'-aminophényl)1,3-diamino 2-propanol.

7. Utilisation selon l'une quelconque des revendications 1 à 5, caractérisée par le fait que l'on utilise également d'autres coupleurs en plus du 4-hydroxy- ou du 4-aminobenzimidazole ou de leurs dérivés, choisis parmi les métadiphénols, les métaaminophénols, les métaacylaminophénols, les métauréidophénols, les métacarbalcoxyaminophénols, les naphtols, les coupleurs à groupement méthylène actif, les pyrazolones et les coupleurs de la famille des indoles.

8. Composition tinctoriale pour fibres kératiniques, en particulier des cheveux humains, caractérisée par le fait qu'elle contient dans un milieu approprié pour la teinture des fibres kératiniques, au moins un coupleur de formule (I) définie dans la revendication 1 et au moins une p-phénylènediamine, le rapport de la p-phénylènediamine/coupleur de formule (I) étant supérieur ou égal à 1,2 et de préférence supérieur ou égal à 1,5.

9. Composition selon la revendication 8, caractérisée par le fait que la p-phénylènediamine répond à la formule (II) définie dans la revendication 2.

10. Composition selon la revendication 8 ou 9, caractérisée par le fait qu'elle contient en outre des coupleurs différents de ceux de formule (I) et/ou des colorants directs.

11. Composition selon l'une quelconque des revendications 8 à 10, caractérisée par le fait que le coupleur de formule (I) est présent dans des proportions comprises entre 0,008 et 3,5% en poids par rapport au poids total de la composition.

12. Composition selon l'une quelconque des revendications 8 à 11, caractérisée par le fait que la p-phénylènediamine est présente dans la composition dans des proportions comprises entre 0,01 et 10% en poids par rapport au poids total de la composition, le rapport p-phénylènediamine/coupleur de formule (I) étant égal ou supérieur à 1,2 et de préférence égal ou supérieur à 1,5.

13. Procédé de coloration des fibres kératiniques, en particulier des cheveux humains, caractérisé par le fait que l'on applique sur les fibres kératiniques une composition telle que définie dans l'une quelconque des revendications 8 à 12, mélangée au préalable avec un agent oxydant, dans des quantités suffisantes pour développer la coloration lors de l'application sur les fibres kératiniques.

14. Procédé de coloration des fibres kératiniques, en particulier des cheveux humains, caractérisé par le fait que l'on applique une composition (A) contenant dans un milieu approprié pour la teinture le coupleur de formule (I) définie dans la revendication 1 et une p-phénylènediamine dans les proportions définies dans les revendications 11 et 12 et qu'après rinçage, on applique une composition (B) contenant l'agent oxydant, le rapport entre la p-phénylènediamine et le coupleur de formule (I) étant supérieur ou égal à 1,2.

15. Procédé de teinture des fibres kératiniques, en particulier des cheveux humains, caractérisé par le fait que l'on applique dans un premier temps, une composition (A) contenant une p-phénylène diamine dans un milieu approprié pour la teinture, et, après rinçage, on applique une composition (B) renfermant dans un milieu approprié pour la teinture, le coupleur de formule (I) définie dans la revendication 1 et l'agent oxydant, ledit coupleur étant présent dans des proportions telles que le rapport p-phénylènediamine/coupleur de formule (I) soit supérieur ou égal à 1,2.

## Claims

1. Use of 4-hydroxy- or 4-aminobenzimidazole or their derivatives corresponding to the formula (I): 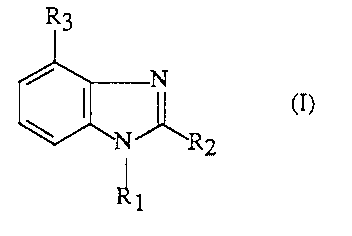 in which:
R₁ and R₂ represent hydrogen or alkyl, one at least of the substituents being equal to hydrogen;
R₃ denotes OH or NH₂,
as coupler for dyeing keratinous fibres, in particular hair, in the presence of a paraphenylenediamine, the ratio between the paraphenylenediamine and the coupler of formula (I) being greater than or equal to 1.2.

2. Use according to Claim 1, characterized in that the paraphenylenediamine is chosen from compounds corresponding to the formula (II): 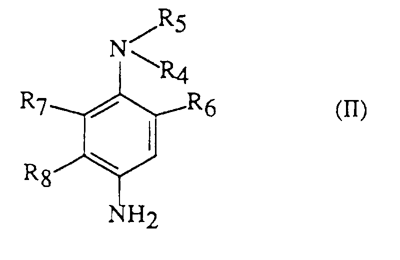 in which:
R₆, R₇ and R₈, which are identical or different, represent a hydrogen or halogen atom, an alkyl or hydroxyalkyl radical having 1 to 4 carbon atoms, an alkoxy radical having 1 to 4 carbon atoms or a carboxyl radical; R₄ and R₅, which are identical or different, represent a hydrogen atom or an alkyl, hydroxyalkyl, alkoxyalkyl, carbamoylalkyl, mesylaminoalkyl, acetylaminoalkyl, sulphoalkyl, ureidoalkyl, carbalkoxyaminoalkyl, piperidinoalkyl, morpholinoalkyl or phenyl radical, these alkyl or alkoxy groups having from 1 to 4 carbon atoms, or else R₄ and R₅ form, jointly with the nitrogen atom to which they are bonded, a piperidino or morpholino heterocycle, with the proviso that R₆ or R₇ represents a hydrogen atom when R₄ and R₅ do not represent a hydrogen atom, and the salts of these compounds.

3. Use according to either of Claims 1 and 2, characterized in that 4-hydroxybenzimidazole is combined with p-phenylenediamine or with p-toluylenediamine.

4. Use according to any one of Claims 1 to 3, characterized in that other oxidation dye precursors of ortho type chosen from orthoaminophenols and orthophenylenediamines and of para type chosen from bisphenylalkylenediamines or para heterocyclic precursors are also used.

5. Use according to Claim 4, characterized in that the so-called "double" bases of the bisphenylalkylenediamine family are chosen from compounds corresponding to the formula (III): 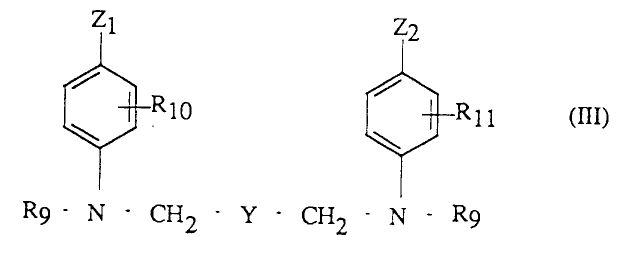 in which:
Z₁ and Z₂, which are identical or different, represent hydroxyl groups or NHR₁₂ groups where R₁₂ denotes a hydrogen atom or a lower alkyl radical;
R₁₀ and R₁₁, which are identical or different, represent either hydrogen atoms or halogen atoms or also alkyl groups;
R₉ represents a hydrogen atom or an alkyl, hydroxyalkyl or aminoalkyl group in which the amino residue may be substituted;
Y represents a radical taken from the group consisting of the following radicals: -(CH₂)ₙ-, (CH₂)ₘ-O-(CH₂)ₘ, 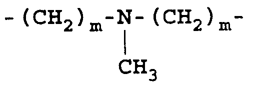 and -(CH₂)ₘ-CHOH-(CH₂)ₘ;
n being an integer between 0 and 8 and m an integer between 0 and 4, it being possible for this base to be provided in the form of its addition salts with acids.

6. Use according to any one of Claims 1 to 5, characterized in that 4-hydroxybenzimidazole is combined with p-phenylenediamine or with p-toluylenediamine and with N,N'-bis(β-hydroxyethyl)-N,N'-bis(4'-aminophenyl)-1,3-diamino-2-propanol.

7. Use according to any one of Claims 1 to 5, characterized in that, in addition to 4-hydroxy- or 4-aminobenzimidazole or their derivatives, other couplers chosen from metadiphenols, metaaminophenols, metaacylaminophenols, metaureidophenols, metacarbalkoxyaminophenols, naphthols, couplers containing an active methylene group, pyrazolones and couplers of the indole family, are also used.

8. Dyeing composition for keratinous fibres, in particular human hair, characterized in that it contains, in a medium suitable for dyeing keratinous fibres, at least one coupler of formula (I) defined in Claim 1 and at least one p-phenylenediamine, the ratio of the p-phenylenediamine/ coupler of formula (I) [sic] being greater than or equal to 1.2 and preferably greater than or equal to 1.5.

9. Composition according to Claim 8, characterized in that the p-phenylenediamine corresponds to the formula (II) defined in Claim 2.

10. Composition according to Claim 8 or 9, characterized in that it additionally contains couplers other than those of formula (I) and/or direct dyes.

11. Composition according to any one of Claims 8 to 10, characterized in that the coupler of formula (I) is present in proportions between 0.008 and 3.5 % by weight with respect to the total weight of the composition.

12. Composition according to any one of Claims 8 to 11, characterized in that the p-phenylenediamine is present in the composition in proportions between 0.01 and 10 % by weight with respect to the total weight of the composition, the p-phenylenediamine/coupler of formula (I) ratio being equal to or greater than 1.2 and preferably equal to or greater than 1.5.

13. Process for colouring keratinous fibres, in particular human hair, characterized in that a composition as defined in any one of Claims 8 to 12, mixed beforehand with an oxidizing agent, is applied to the keratinous fibres in amounts sufficient to develop the colouring during application to keratinous fibres.

14. Process for colouring keratinous fibres, in particular human hair, characterized in that a composition (A) containing, in a medium suitable for dyeing, the coupler of formula (I) defined in Claim 1 and a p-phenylenediamine in the proportions defined in Claims 11 and 12 is applied and in that, after rinsing, a composition (B) containing the oxidizing agent is applied, the ratio between the p-phenylenediamine and the coupler of formula (I) being greater than or equal to 1.2.

15. Process for dyeing keratinous fibres, in particular human hair, characterised in that, in a first step, a composition (A) containing a p-phenylenediamine in a medium suitable for dyeing is applied and, after rinsing, a composition (B) containing, in a medium suitable for dyeing, the coupler of formula (I) defined in Claim 1 and the oxidizing agent is applied, the said coupler being present in proportions such that the p-phenylenediamine/coupler of formula (I) ratio is greater than or equal to 1.2.

## Patentansprüche

1. Verwendung von 4-Hydroxy- oder von 4-Aminobenzimidazol oder von deren Derivaten, welche die Formel (I) aufweisen: 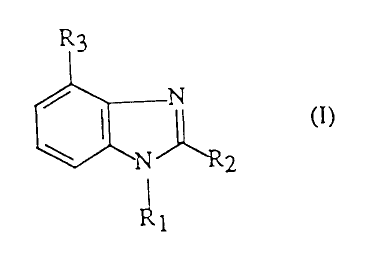 worin gilt:
R₁ und R₂ stellen Wasserstoff oder einen Alkylrest dar, wobei mindestens einer der Substituenten Wasserstoff ist;
R₃ bedeutet OH oder NH₂,
als Kuppler zur Färbung keratinischer Fasern, insbesondere der Haare, in Gegenwart eines p-Phenylendiamin, wobei das Verhältnis zwischen dem p-Phenylendiamin und dem Kuppler der Formel (I) höher oder gleich 1,2 ist.

2. Verwendung gemäß Anspruch 1,
dadurch **gekennzeichnet**, daß
das p-Phenylendiamin aus Verbindungen der Formel (II): 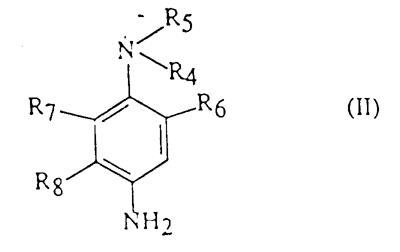 worin gilt:
R₆, R₇ und R₈ stellen, gleich oder verschieden, ein Wasserstoff- oder Halogenatom, einen Alkyl- oder Hydroxyalkylrest mit 1 bis 4 Kohlenstoffatomen, einen Alkoxyrest mit 1 bis 4 Kohlenstoffatomen oder einen Carboxyrest und R₄ und R₅ stellen, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Sulfoalkyl-, Ureidoalkyl-, Carbalkoxyaminoalkyl-, Piperidinoalkyl-, Morpholinoalkyl- und einen Phenylrest dar, wobei die jeweiligen Alkyl- oder Alkoxygruppen 1 bis 4 Kohlenstoffatome aufweisen, oder R₄ und R₅ bilden, zusammen mit dem Stickstoffatom, an das sie gebunden sind, auch einen Piperidino- oder Morpholino-Heterozyklus, mit der Maßgabe, daß R₇ oder R₆ ein Wasserstoffatom darstellen, wenn R₄ und R₅ kein Wasserstoffatom darstellen,
sowie aus Salzen dieser Verbindungen ausgewählt ist.

3. Verwendung gemäß Anspruch 1 oder 2,
dadurch **gekennzeichnet**, daß
man das 4-Hydroxybenzimidazol mit p-Phenylendiamin oder p-Toluylendiamin zusammenbringt.

4. Verwendung gemäß einem der Ansprüche 1 bis 3,
dadurch **gekennzeichnet**, daß
man auch weitere Oxidationsfarbstoff-Vorstufenverbindungen vom o-Typ, ausgewählt aus o-Aminophenolen und o-Phenylendiaminen, und vom p-Typ verwendet, ausgewählt aus Bisphenylalkylendiaminen und heterozyklischen p-Vorstufenverbindungen.

5. Verwendung gemäß Anspruch 4,
dadurch **gekennzeichnet**, daß
die als "doppelte" bezeichneten Basen der Familie der Bisphenylalkylendiamine aus Verbindungen der Formel (III) ausgewählt sind: 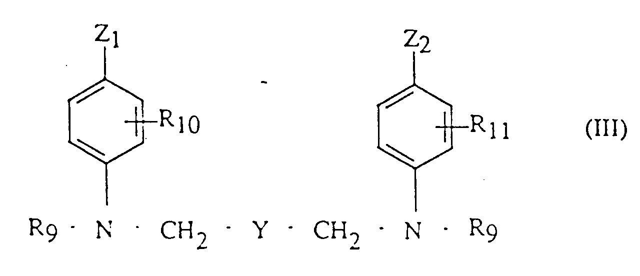 worin gilt:
Z₁ und Z₂ stellen, gleich oder verschieden, Hydroxyl- oder NHR₁₂-Gruppierungen dar, worin R₁₂ ein Wasserstoffatom oder einen Niedrigalkylrest bedeutet;
R₁₀ und R₁₁ stellen, gleich oder verschieden, entweder Wasserstoff- oder Halogenatome oder auch Alkylgruppen dar;
R₉ stellt ein Wasserstoffatom, eine Alkyl-, Hydroxyalkyl- oder Aminoalkylgruppe dar, worin der Aminorest substituiert sein kann;
y stellt einen Rest aus der Gruppe aus den folgenden Resten dar: -(CH₂)ₙ-, (CH₂)ₘ-O-(CH₂)ₘ, -(CH₂)ₘ-N-CH₃-(CH₂)ₘ-; -(CH₂)ₘ-CHOH-(CH₂)ₘ,
worin n eine ganze Zahl von 0 bis 8 und m eine ganze Zahl von 0 bis 4 sind,
wobei diese Base in Form ihrer Additionssalze mit Säuren vorliegen kann.

6. Verwendung gemäß einem der Ansprüche 1 bis 5,
dadurch **gekennzeichnet**, daß
man das 4-Hydroxybenzimidazol mit p-Phenylendiamin oder p-Toluylendiamin und mit N,N'-Bis(β-hydroxyethyl)-N,N'-bis(4'-aminophenyl)-1,3-diaminoproan-2-ol zusammenbringt.

7. Verwendung gemäß einem der Ansprüche 1 bis 5,
dadurch **gekennzeichnet**, daß
man weitere Kuppler zusätzlich zum 4-Hydroxy- oder 4-Aminobenzimidazol oder zu den Derivaten verwendet, ausgewählt aus m-Diphenolen, m-Aminophenolen, m-Acylaminophenolen, m-Ureidophenolen, m-Carbalkoxyaminophenolen, Naphtholen, Kupplern mit aktiver Methylengruppe, Pyrazolonen und aus Kupplern der Familie der Indole.

8. Färbezusammensetzung für keratinische Fasern, insbesondere für menschliche Haare,
dadurch **gekennzeichnet**, daß
sie in einem zur Färbung der keratinischen Fasern geeigneten Medium mindestens einen Kuppler der im Anspruch 1 definierten Formel (I) und midnestens ein p-Phenylendiamin enthält, wobei das Verhältnis von p-Phenylendiamin/Kuppler der Formel (I) höher oder gleich 1,2 und vorzugsweise höher oder gleich 1,5 ist.

9. Zusammensetzung gemäß Anspruch 8,
dadurch **gekennzeichnet**, daß
das p-Phenylendiamin die im Anspruch 2 definierte Formel (II) aufweist.

10. Zusammensetzung gemäß Anspruch 8 oder 9,
dadurch **gekennzeichnet**, daß
die Zusammensetzung zusätzlich Kuppler, die sich von denen der Formel (I) unterscheiden, und/oder Direktfarbstoffe enthält.

11. Zusammensetzung gemäß einem der Ansprüche 8 bis 10,
dadurch **gekennzeichnet**, daß
der Kuppler der Formel (I) in Mengenanteilen von 0,008 bis 3,5 Gew.% vorhanden ist, bezogen auf das Gesamtgewicht der Zusammensetzung.

12. Zusammensetzung gemäß einem der Ansprüche 8 bis 11,
dadurch **gekennzeichnet**, daß
das p-Phenylendiamin in der Zusammensetzung in Mengenanteilen von 0,01 bis 10 Gew.% vorhanden ist, bezogen auf das Gesamtgewicht der Zusammensetzung, wobei das Verhältnis von p-Phenylendiamin/Kuppler der Formel (I) gleich oder höher 1,2 und vorzugsweise gleich oder höher 1,5 ist.

13. Verfahren zur Färbung keratinischer Fasern, insbesondere der menschlichen Haare,
dadurch **gekennzeichnet**, daß
man auf die keratinischen Fasern eine in jedem der Ansprüche 8 bis 12 definierte Zusammensetzung, die vorab mit einem oxidierenden Mittel vermischt worden ist, in hinreichenden Mengen aufbringt, um die Färbung während der Aufbringung auf den keratinischen Fasern zu entwickeln.

14. Verfahren zur Färbung keratinischer Fasern, insbesondere der menschlichen Haare,
dadurch **gekennzeichnet**, daß
man eine Zusammensetzung (A), die in einem zur Färbung geeigneten Medium den Kuppler der in Anspruch 1 definierten Formel (I) und ein p-Phenylendiamin in den in den Ansprüchen 11 oder 12 definierten Anteilsmengen enthält, und nach Spülung eine Zusammensetzung (B) aufbringt, die ein oxidierendes Mittel enthält, wobei das Verhältnis zwischen dem p-Phenylendiamin und dem Kuppler der Formel (I) höher oder gleich 1,2 ist.

15. Verfahren zur Färbung keratinischer Fasern, insbesondere der menschlichen Haare,
dadurch **gekennzeichnet**, daß
man in einer ersten Stufe eine Zusammensetzung (A), die ein p-Phenylendiamin in einem zur Färbung geeigneten Medium enthält, und, nach Spülung, eine Zusammensetzung (B) aufbringt, die in einem zur Färbung geeigneten Medium den Kuppler der in Anspruch 1 definierten Formel (I) und ein oxidierendes Mittel umfaßt, wobei der genannte Kuppler in solchen Mengenanteilen vorhanden ist, daß das Verhältnis von p-Phenylendiamin/Kuppler der Formel (I) höher oder gleich 1,2 ist.
